# EUROPEAN PATENT APPLICATION

(11) **EP 3 669 671 A1**
(43) Date of publication of application: **24.06.2020**
(21) Application number: 18214230.7
(22) Date of filing: 19.12.2018
(51) Int. Cl.: A23L 33/00, A23C 9/20, A61K 31/7034, C07H 1/08, C07H 13/04, A23L 33/21

(54) **A PROCESS TO ENRICH OLIGOSACCHARIDES FROM WHEY STREAMS AND A COMPOSITION THEREOF**

(71) Applicant: Agriculture and Food Development Authority (TEAGASC), Co. Carlow (IE)
(72) Inventor: MAROTTA, Mariarosaria, 83100 Avellino (IT); HICKEY, Rita, Clonmel, Co. Tipperary (IE)
(74) Representative: Purdylucey Intellectual Property

(57) **Abstract**

A method for generating a lactose-based oligosaccharide-enriched fraction with a desirable concentration of siallylactose, low concentrations of ash and non-protein nitrogen content, and substantially free from protein, the method comprising the steps of applying a solution comprising siallylactose to ion exclusion chromatography, where the resin is a strong cationic exchange resin, in which the resin is in a divalent form.

## Description

### Field of the Invention

The invention relates to a process for generating lactose-based, complex-enriched oligosaccharides.

### Background to the Invention

Breast milk is the sole food that nature has selected to nourish our babies. The World Health Organisation recommends exclusively breastfeeding babies for the first 6 months of their life. Mother's milk not only contains all the components necessary for the growth and development of the babies, but also contains components that are essential to promote and maintain their health. Although breastfeeding is the first choice for the new-born baby, when this is not possible, infant formulas play a key role in infant nutrition. As a result of this role, there is worldwide interest in designing milk infant formulas that increasingly resemble mother's milk. Recent research all over the world is focusing on matching the composition and functional outcome of human milk looking at the amino acid profile, the fatty acid content and the adaptation of the triacylglycerol structure of fat, and also the oligosaccharides fraction.

Breastfed babies are known to be less prone to infections when compared to formula fed infants. Recent studies conducted by researchers across the world indicate that human milk oligosaccharides (HMO), which make up the third largest component in breast milk, can act as prebiotics by promoting the growth of health promoting bacteria in the babies' gut, prevent infections, boost immunity and aid in brain development.

By contrast, oligosaccharides, while present, are found in very low concentrations in bovine milk and, consequently, in infant formulas. Most of the infant formulas on the market are based on bovine milk, which means that if some components are not present in bovine milk or are present only in trace amounts, they cannot be found in infant formulas unless they are subsequently added. This is exactly the case for oligosaccharides. In recent years, infant formula manufacturers have been adding galactooligosaccharides and fructooligosaccharides as substitutes for HMO. However, these ingredients do not have the same complexity of HMO, which suggests that they are unlikely to confer all the health benefits associated with milk oligosaccharides. For this reason, there is an increasing interest in finding oligosaccharides which are closer to those found in human milk. In this respect, many companies are synthesising individual milk oligosaccharides, which can be added to infant formulas on their own or in combination. An alternative approach is the possibility of extracting and enriching oligosaccharides from bovine milk. Although the concentration of oligosaccharides in bovine milk is low, recent studies have shown that bovine milk oligosaccharides (BMO) share some structural similarities with HMO, which may imply shared functionalities. This would suggest that BMO may act as a potential substitute for HMO, providing formula fed infants with similar health benefits to that of breastfed infants.

EP2094366 describes a method for purifying sialylactose (SL) by ion exclusion chromatography from a solution containing SL using a strong cationic resin in predominantly monovalent form, Na or K, and for obtaining a raffinate enriched in SL. The method comprises a preliminary step for separating all or part of the calcium phosphate. The method employs a cation exchange resin in monovalent form, to provide a product that contains ∼1.2% sialyllactose (SL), ∼40% lactose and ∼57% remainder (it is not stated what this remainder is made up of). It is very unlikely that it can be used for applications in infant nutrition. EP2408311 describes a process for the demineralisation and fractionation of whey or raw milk with minimal or no use of chemicals and wherein the source of the whey or milk has a reduced bearing on the cost of the overall process. Most studies in this area focus on enrichment and recovery of sialylated oligosaccharides from dairy streams, especially patents that use anion exchange chromatography. However, the resulting products generally were unsuitable for use in infant milk formula.

It is an aim of the subject application to overcome at least one of the above-referenced problems.

### Summary of the Invention

As milk oligosaccharides have been found also in whey streams, the inventors investigated processes for enriching BMO from whey streams, which are low cost products compared to other dairy streams. To evaluate enrichment of BMO throughout the process, sialyllactose (SL) was selected as a marker of total BMO, since it is the predominant oligosaccharide in the BMO pool and can be quantified by using routine analytical methods.

There is provided, as set out in the appended claims, a method for generating a lactose-based complex oligosaccharide enriched fraction with reduced proteins, peptides and ash, the method comprising the following steps:
- providing a demineralised dairy stream;
- optionally removing undesirable proteins by applying the demineralised dairy stream to an adsorbent resin; and
- applying the demineralised dairy stream to ion exclusion chromatography, in which the resin is a divalent, cation exchange resin, the oligosaccharide-enriched fraction being eluted through ion exclusion chromatography.

It should be noted that the demineralised dairy stream is applied to a strong divalent cation exchange resin which is used in an ion exclusion chromatographic mode. The eluate is collected through ion exclusion rather than cation exchange. When cation-exchange resins are used in an ion-exclusion mode, there is no exchange of cations. In the examples described herein, deionised water was used to elute the oligosaccharide-enriched fraction.

There is provided, in an aspect, a method for generating a lactose-based oligosaccharide-enriched fraction with an increased concentration of siallylactose relative to milk and standard dairy streams, low concentrations of ash and non-protein nitrogen content, and substantially free from protein, the method comprising the steps of (a) providing a dairy stream comprising siallylactose and (b) applying the dairy stream to ion exclusion chromatography using a divalent cation exchange resin and (c) collecting eluate containing siallylactose and lactose to generate the lactose-based oligosaccharide-enriched fraction.

In an aspect, the method is carried out at a temperature between room temperature and 80 °C. Preferably, the method is carried out a temperature of 60°C.

In an aspect, the dairy stream comprising siallylactose is selected from a commercial milk by-product that is substantially free of casein and retains lactose and oligosaccharides. Preferably, the dairy stream is selected from demineralised whey, demineralised whey permeate, whey permeate, or mother liquor.

In an aspect, when the dairy stream selected is not demineralised whey permeate, an additional step of applying the dairy stream to an adsorbent resin is performed after step (a) and before step (b) to deplete the dairy stream of proteins.

In an aspect, the lactose-based oligosaccharide-enriched fraction has a concentration of 0.5 to 1.5% siallylactose/total solids, lactose 88-95%, proteins 0%, non-protein nitrogen (NPN) up to 0.5%, and ash up to 0.6%. Preferably, the lactose-based oligosaccharide-enriched fraction has a concentration of 0.7-1.5% (dry weight) siallylactose/total solids.

In an aspect, the lactose-based oligosaccharide-enriched fraction is in a dried form.

In an aspect there is provided, as set out in the appended claims, a lactose-based oligosaccharide-enriched composition containing 88-95% lactose (w/w), 0.5-1.5% sialyllactose (w/w), 0.1-0.5% non-protein nitrogen (NPN) (w/w), and 0.1-0.6% ash (w/w).

In an aspect, there is provided a lactose-based oligosaccharide-enriched composition produced by the method described above that is substantially free of casein and retains lactose and oligosaccharides, the composition comprising sialyllactose at a concentration of between 0.5% and 1.5% (w/w) of total solids and which is substantially free of protein.

In an aspect, the composition comprises up to 0.5% (w/w) NPN and up to 0.6% (w/w) ash.

The composition is preferably in dry form, for example a particulate product such as a powder, flakes, pellets and such like, but can also be in wet form. The dry composition may be generated by means of any suitable drying technique, such as spray drying, drum drying or freeze-drying. The invention also relates to liquid compositions comprising the composition of the invention suspended or solubilised in a suitable liquid such as water (sterilised, purified, deionised, or a combination thereof).

The invention also provides a food product comprising the milk-derived, oligosaccharide enriched composition of the invention. Typically, the food product is selected from a dairy product, a beverage, infant food, a cereal, a biscuit, confectionary, a cake, a food supplement, a dietary supplement, an animal feed, a poultry feed. Typically, the food product comprises 0.1-10.0%, or 0.1-5.0%, or 0.1-1.0% (w/w) of the oligosaccharide-enriched composition of the invention.

In an aspect, there is provided a food product comprising a lactose-based oligosaccharide-enriched fraction produced by the method or using a composition described above.

In an aspect, the food product is an infant formula. Preferably, the lactose-based oligosaccharide-enriched fraction makes up from 0.1 to 10.0% (w/v) of the infant formula.

In an aspect, the food product is selected from a dairy product, a beverage, infant food, a cereal, a biscuit, confectionary, a cake, a food supplement, a dietary supplement, an animal feed, and a poultry feed.

The invention also relates to an infant formula comprising a milk derived oligosaccharide enriched composition according to the invention, typically in an amount of 0.1 to 10.0% (w/v), preferably about 1 to 7.5% (w/v), and ideally about 2.0 to 4.0% (w/v) of reconstituted formula.

The composition of the invention is derived from a commercial milk by-product that is substantially free of casein and retains lactose and oligosaccharides, for example demineralised whey and whey permeate, whey permeate, or mother liquor.

### Definitions

The term "milk derived" should be understood to mean that the composition is obtained from commercial milk sources or by-products, typically obtained from a whey fraction, for example whey permeate, mother liquor, demineralised whey or demineralised whey powder. In a preferred embodiment, the composition is derived from a commercial bovine milk whey fraction. The term "milk" should be primarily understood to mean bovine milk, but may also include milk from other mammals such as buffalo, camel, alpaca, sheep, donkey or goats. Commercial milk sources mean mature milks commonly employed in commerce.

Preferably, substantially all of the oligosaccharides in the composition or fraction, for example at least 85%, 87%, 90%, 92%, 95% or 97% of the total detectable oligosaccharide, are oligosaccharides ranging in size from 300-1200 Da as determined by the method outlined by Marino et al. (2011) Glycobiology 21; 1317-1330.

The term "substantially free" as applied to protein means that less than 1% (dry weight), and preferably less than 0.1% (dry weight) of the composition comprises protein having a molecular weight of greater than 1kDa. Ideally, the composition is free of protein having a molecular weight of greater than 1kDa.

In this specification, the term "oligosaccharide-enriched composition" or "oligosaccharide-enriched fraction" should be understood to mean a composition or fraction comprising at least 0.5 %, 0.7%, 0.9%, 1.1%, 1.4%, 1.5%, 1.6%, 1.7%, 1.8%, 1.9%, 2.0%, 2.5%, 3.0%, 3.5%. 4.0%, 4.5% or 5.0% oligosaccharides (w/w) without an increase in ash or NPN levels above 0.1-0.6% and 0.1-0.5%, respectively. In an aspect, the composition or fraction comprises no more than 1.5% oligosaccharides (w/w).

In the specification, the term "low in peptides" means less than 1 g/L peptides.

In this specification, the term "dairy product stream" should be understood to mean a dairy product stream obtained from commercial milk which retains lactose and oligosaccharides and from which caseins have been removed. Preferably, the dairy product stream is whey fraction dairy product stream. Examples include whey permeate, mother liquor, demineralised whey permeate and other dairy streams obtained using demineralised whey.

In the specification, the percentages provided to the components of the lactose-based oligosaccharide-enriched fraction and composition comprising the same relate to percent (w/w), that is, the percentage of that component in the total amount of total solids.

In the specification, the term "room temperature" should be understood to mean ambient temperature of between 18°C and 25°C, that is, 18°C, 19°C, 20°C, 21°C, 22°C, 23°C, 24°C or 25°C.

### Detailed Description

The inventors have developed processes that, using either sequential chromatographic steps or a single chromatographic step when starting from demineralized whey streams, generated a lactose-based complex oligosaccharide enriched fraction (OSF), which was reduced in proteins/peptides and ash. This ingredient has a concentration of 0.7-1.5% SL/total solids, lactose 88-95%, proteins 0%, non-protein nitrogen (NPN) up to 0.5%, ash up to 0.6%; and when combined with other infant formula ingredients, will result in a concentration of 0.03 g SL/100mL as found in human milk. Furthermore, other oligosaccharides are expected to be found in the OSF.

Demineralisation is essential for the generation of a high quality OSF which can be used as an ingredient for a number of nutritional and biomedical applications as multiple biological activities are attributed to milk oligosaccharides e.g. prebiotic effects, they act as receptors analogues to inhibit the adhesion of pathogens to the epithelial surface and interact directly with immune system. Milk oligosaccharides may also play a role in postnatal brain development. The demineralised streams will be applied to a divalent cation exchange resin, which can be preceded by an adsorbent resin to remove any residual protein. The steps of the process includes the use of two resins: 1) an adsorbent resin (AR) to remove proteins and 2) a divalent cation exchange resin (CEX) used in an ion exclusion chromatography mode, to reduce lactose.

The process of the invention generates a lactose-based complex oligosaccharides fraction, which can be used as is; there is no need for further processing of this OSF when adding to infant formula. Most of other technologies produced ingredients that, although enriched in acidic oligosaccharides, were not balanced in terms of all the components in it for application.

### Materials and Methods

### Enrichment of oligosaccharides

For enrichment of OS, demineralised whey permeate powder was re-suspended in water at 18% total solids. 10 mL of this solution was applied to a divalent cation exchange resin based on sulfonated poly(styrene-divinylbenzene) supports (UBK535 from Mitsubishi; Purolite PCR642) charged with Ca²⁺ (2.6 x 88 cm) kept at 60°C and eluted with deionized water at a flow rate of 2.0 mL min⁻¹. The fractions were eluted in ion exclusion mode. The fractions (6mL) were analysed for 3-SL and 6-SL using High pH Anion Exchange Chromatography with Pulsed Amperometric Detection (HPAEC-PAD). Fractions containing sialyllactose (SL) and lactose at a concentration ≤ 25g/L were pooled to give an oligosaccharide-enriched fraction (OSF). The resulting OSF has the right combination of all the components required for use in a food product such as infant formula.

However, if a dairy stream is used which is richer in protein than, for example, whey permeates, an adsorbent resin step can be introduced prior to the CEX step to deplete the dairy stream of proteins. Typical ARs include Sepabeads SP850, Sepabeads SP207, Diaion HP2MGL, and Diaion HP20 (all Mitsubishi Chemical).

### Quantification of lactose and sialyllactose by HPLC

Demineralised whey permeate powder, fractions from CEX and OSF were appropriately diluted in water and analysed for quantification of lactose, 3-SL and 6-SL. Lactose in demineralised whey permeate powder, fractions from CEX chromatography and oligosaccharide-enriched product was quantified by HPLC using an HPX-87C Carbohydrate column (300 x 7.8 mm) (Aminex, Bio-Rad, UK) and a refractive index detector. The elution was obtained in isocratic conditions using 4.5 mM sulphuric acid for 30 min. 3-SL and 6-SL in all samples were quantified by HPAEC-PAD, according to Mehra et al. (PLOS ONE (2014): 8;9(5):e96040).

### Structural characterisation of milk oligosaccharides

The free OS in the OSF were structurally characterised by Hydrophobic Interaction Liquid Chromatography (HILIC) as described by Marino *et al.* (2011).

### Results

The OSF obtained from the divalent cation exchange chromatography was characterised by a concentration of 1.35% (w/w) SL, 92% (w/w) lactose, 0% (w/w) proteins, 0.3% (w/w) NPN, ash 0.5% (w/w) with a recovery of 71% of initial SL. The oligosaccharide profile of the OSF was analysed. Analysis of OSF by HILIC highlighted the presence of at least 10 other OS, most of which were sialylated OS.

**Table 1: Composition of demineralised whey permeate and OSF**

| **Sample** | **SL** | **Lactose** | **Protein** | **NPN** | **Ash** |
|---|---|---|---|---|---|
| | **as %TS** | | | | |
| Demineralised whey permeate | 0.166 | 98.6 | 0.15 | 0.2 | 0.43 |
| OSF | 1.36 | 92.1 | 0 | 0.29 | 0.50 |

By slightly reducing lactose in OSF, the inventors noted that there was a greater increase in SL without substantially affecting NPN and Ash levels. This finding is important as there has been a greater emphasis in keeping the concentrations of Ash and NPN levels low when the composition is being used in infant formula.

**Table 2: Composition of demineralised whey permeate and OSF following the two-step chromatographic process**

| **Sample** | **SL** | **Lactose** | **Proteins** | **NPN** | **Ash** |
|---|---|---|---|---|---|
| | **as %TS** | | | | |
| **Demineralised whey permeate** | 0.157 | 98.8 | 0.15 | 0.2 | 0.21 |
| **Sample from AR** | 0.143 | 88.9 | 0 | 0.18 | 0.28 |
| **OSF** | 0.75 | 90.2 | 0 | 0 | 0.31 |

When AR was used as an intermediate step when the starting material was demineralised whey permeate, the characteristics of the OSF were in keeping with the desired parameters of the OSF (desirable concentrations of SL and Lactose, and zero or low concentrations of proteins, NPN and Ash).

**Table 3: Recovery of the components after applying demineralised whey permeate on a Ca2+ cation exchange resin**

| | **Recovery (%)** | | | | |
|---|---|---|---|---|---|
| **Sample** | **SL** | **Lactose** | **Protein** | **NPN** | **Ash** |
| Demineralised whey permeate | - | - | - | - | - |
| OSF | 70.9 | 8.10 | 0 | 46 | 9.94 |

One of the advantages of the invention is that an OSF was provided with a right proportion of all the components (SL, lactose, NPN, ash) that would allow the addition of this ingredient in infant formulas without further processing. Having generated a lactose-based ingredient, this has the advantage of not increasing undesired components such as NPN and ash, which is usually observed when most of the lactose component is removed, which consequently requires further steps to reduce/remove the undesired components. The process described herein avoids the need for this additional step.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A method for generating a lactose-based oligosaccharide-enriched fraction with an increased concentration of siallylactose relative to milk and standard dairy streams, low concentrations of ash and non-protein nitrogen content, and substantially free from protein, the method comprising the steps of (a) providing a dairy stream comprising siallylactose and (b) applying the dairy stream to ion exclusion chromatography using a divalent cation exchange resin and (c) collecting eluate containing siallylactose and lactose to generate the lactose-based oligosaccharide-enriched fraction.

2. The method of Claim 1, wherein the method is carried out at a temperature between room temperature and 80°C.

3. The method of Claim 2, wherein the method is carried out a temperature of 60°C.

4. The method of any one of Claims 1 to 3, wherein the dairy stream comprising siallylactose is selected from a commercial milk by-product that is substantially free of casein and retains lactose and oligosaccharides.

5. The method of Claim 4, wherein the dairy stream is selected from demineralised whey, demineralised whey permeate, whey permeate, or mother liquor.

6. The method of any one of the preceding claims, wherein when the dairy stream selected is not demineralised whey permeate, an additional step of applying the dairy stream to an adsorbent resin is performed after step (a) and before step (b) to deplete the dairy stream of proteins.

7. The method according to any one of the preceding claims, wherein the lactose-based oligosaccharide-enriched fraction has a concentration of 0.5 to 1.5% siallylactose/total solids, lactose 88-95%, proteins 0%, non-protein nitrogen (NPN) up to 0.5%, and ash up to 0.6%.

8. The method according Claim 8, in which the lactose-based oligosaccharide-enriched fraction has a concentration of 0.7-1.5% (dry weight) siallylactose/total solids.

9. The method according to any one of the preceding claims, wherein the lactose-based oligosaccharide-enriched fraction is in a dried form.

10. A lactose-based oligosaccharide-enriched composition produced by the method of Claim 1 that is substantially free of casein and retains lactose and oligosaccharides, the composition comprising siallylactose at a concentration of between 0.5% and 1.5% (w/w) of total solids and which is substantially free of protein.

11. The lactose-based oligosaccharide-enriched composition according to Claim 10, wherein the composition comprises up to 0.5% (w/w) NPN and up to 0.6% (w/w) ash.

12. A food product comprising a lactose-based oligosaccharide-enriched fraction produced by the method of Claim 1 or a composition according to Claim 11.

13. The food product according to Claim 12, in which the food product is an infant formula.

14. The food product according to Claim 13, wherein the lactose-based oligosaccharide-enriched fraction makes up from 0.1 to 10.0% (w/v) of the infant formula.

15. The food product according to Claim 12, in which the food product is selected from a dairy product, a beverage, infant food, a cereal, a biscuit, confectionary, a cake, a food supplement, a dietary supplement, an animal feed, and a poultry feed.
